# EUROPEAN PATENT SPECIFICATION

(11) **EP 4 294 485 B1**
(45) Date of publication and mention of the grant of the patent: **08.04.2026**
(21) Application number: 22707686.6
(22) Date of filing: 21.02.2022
(51) Int. Cl.: A61M 16/00, A61B 5/08, A61B 5/00, G16H 50/30

(54) **PRESSURE SUPPORT DEVICE**
DRUCKUNTERSTÜTZUNGSVORRICHTUNG
DISPOSITIF DE SUPPORT DE PRESSION

(30) Priority: 22.02.2021 US 202163151824 P
(43) Date of publication of application: 27.12.2023
(73) Proprietor: Koninklijke Philips N.V., 5656 AG Eindhoven (NL)
(72) Inventor: MORGAN, Stephan Daniel, 5656 AG Eindhoven (NL); UZONYI, Adrienne K. Fazio, 5656 AG Eindhoven (NL); CATALANO, Thomas, 5656 AG Eindhoven (NL); WINSKI, Jeffrey Ronald, 5656 AG Eindhoven (NL)
(74) Representative: Philips Intellectual Property & Standards
(86) International application number: PCT/EP2022/054170
(87) International publication number: WO 2022/175503

(56) References cited:
- WO-A1-2018/115216
- WO-A1-2020/136035

## Description

### BACKGROUND OF THE INVENTION

### 1. Field of the Invention

The present invention pertains to pressure support devices employing methods of providing a pressure support therapy to a patient.

### 2. Description of the Related Art

Many individuals suffer from disordered breathing during sleep. Sleep apnea is a common example of such sleep disordered breathing suffered by millions of people throughout the world. One type of sleep apnea is obstructive sleep apnea (OSA), which is a condition in which sleep is repeatedly interrupted by an inability to breathe due to an obstruction of the airway; typically the upper airway or pharyngeal area. Obstruction of the airway is generally believed to be due, at least in part, to a general relaxation of the muscles which stabilize the upper airway segment, thereby allowing the tissues to collapse the airway. Another type of sleep apnea syndrome is a central apnea, which is a cessation of respiration due to the absence of respiratory signals from the brain's respiratory center. An apnea condition, whether OSA, central, or mixed, which is a combination of OSA and central, is defined as the complete or near cessation of breathing, for example a 90% or greater reduction in peak respiratory air-flow.

Those afflicted with sleep apnea experience sleep fragmentation and complete or nearly complete cessation of ventilation intermittently during sleep with potentially severe degrees of oxyhemoglobin desaturation. These symptoms may be translated clinically into extreme daytime sleepiness, cardiac arrhythmias, pulmonaryartery hypertension, congestive heart failure and/or cognitive dysfunction. Other consequences of sleep apnea include right ventricular dysfunction, carbon dioxide retention during wakefulness, as well as during sleep, and continuous reduced arterial oxygen tension. Sleep apnea sufferers may be at risk for excessive mortality from these factors as well as by an elevated risk for accidents while driving and/or operating potentially dangerous equipment.

Even if a patient does not suffer from a complete or nearly complete obstruction of the airway, it is also known that adverse effects, such as arousals from sleep, can occur where there is only a partial obstruction of the airway. Partial obstruction of the airway typically results in shallow breathing referred to as a hypopnea. A hypopnea is typically defined as a 50% or greater reduction in the peak respiratory airflow. Other types of sleep disordered breathing include, without limitation, upper airway resistance syndrome (UARS) and vibration of the airway, such as vibration of the pharyngeal wall, commonly referred to as snoring. Thus, in diagnosing a patient with a breathing disorder, such as OSA, central apneas, or UARS, it is important to detect accurately the occurrence of apneas and hypopneas of the patient.

It is well known to treat sleep disordered breathing by applying a positive airway pressure (PAP) to the patient's airway using an airway pressure support system that typically includes a mask, a pressure generating device, and a conduit to deliver positive pressure breathing gas from the pressure generating device to the patient through the mask. This positive pressure effectively "splints" the airway, thereby maintaining an open passage to the lungs. In one type of PAP therapy, known as continuous positive airway pressure (CPAP), the pressure of gas delivered to the patient is constant throughout the patient's breathing cycle. It is also known to provide a positive pressure therapy in which the pressure of gas delivered to the patient varies with the patient's breathing cycle, or varies with the patient's effort, to increase the comfort to the patient. This pressure support technique is referred to as bi-level pressure support, in which the inspiratory positive airway pressure (IPAP) delivered to the patient is higher than the expiratory positive airway pressure (EPAP).

Today in CPAP delivery to patients, prescriptions are written as such that a patient is prescribed a fixed CPAP pressure (constant delivery of a therapeutic pressure) or an Auto CPAP with additional prescribed minimum and maximum pressures so that an auto algorithm can operate within the bounds of the set pressure range. It is not uncommon for patients, especially those new to CPAP therapy, to feel discomfort with their prescribed pressure when falling asleep. Often times this pressure is too high. To address this discomfort, some CPAP providers offer a feature called Ramp or Fixed Ramp. When activated, such feature allows patients to drop their delivered pressure down to 4cmH2O up to their prescribed CPAP pressure or prescribed Auto minimum pressure. This lower Ramp pressure is set/adjusted by the caregiver during an initial and/or return visit(s) until such pressure is at an optimal level desired by the patient. Alternatively, in some versions the pressure can be set by the patient prior to starting a pressure treatment (i.e., when the pressure generating device is not running). The pressure delivered starts at the set Ramp pressure and begins to "ramp" up and increase (linearly) the delivered pressure for a set period of time to the prescribed pressure. Once the set period of time has concluded, the device delivers the fixed pressure (CPAP) or goes back into Auto mode and titrates to the appropriate pressure level.

In devices such as the Philips DreamStation that also offer SMART Ramp, the initial behavior is the same as explained above for Ramp. However, the device uses event detection during the Ramp period to identify if a patient has had an event requiring more pressure than what is being delivered during Ramp. In such instance, the device's auto algorithm kicks in and increases delivered pressure as needed so as not to allow the patient to experience unnecessary events.

Patients generally are allowed to set their own Ramp pressure and Ramp time (usually between 5 and 45 minutes in 5 minute increments). Such selecting/setting of the pressure/time is accomplished in a preliminary step when the device is not yet producing/providing positive pressure breathing gas (i.e therapy is not running). In a CPAP device manufactured and distributed by Philips, there is a dedicated Ramp button that allows the patient to tap each night to activate Ramp, thus dropping the delivered pressure to the lower Ramp pressure.

In the aforementioned Ramp features the bounds of Ramp pressure available to patients are limited, i.e., from 4cmH2O to fixed CPAP pressure or min Auto pressure. For example, if a patient is prescribed an Auto device set from 5cmH2O (min) to 20 cmH2O (max), the possible Ramp pressures the patient can select from are only 4cmH2O or 4.5cmH2O or 5cmH2O. The 5-20cmH2O Auto prescription is the most commonly prescribed. In this case the device will start delivering therapy at a pressure of 5cmH2O if Ramp is OFF or a pressure of say 4cmH2O if Ramp is ON. This might be satisfactory if 5cmH2O was too high for a patient and felt uncomfortable. However, if a patient felt air starved or felt like they were suffocating at 5cmH2O, there is no option to increase the pressure.

In a Philips assessment of patient therapy trends, we have seen that 25% of the time patients are getting an adjustment to their prescribed minimum pressure (when using an Auto mode) in the first 90 days of therapy. Over 85% of these adjustments result in an increase of the minimum prescribed pressure. This suggests that patients are either uncomfortable at lower pressure settings or that a physician may feel like there is a more optimal minimum pressure for treating events.

The current limitations of devices do not allow patients to select an actual starting pressure that is most comfortable for them to fall asleep to. If the pressure is too low and they feel air starved, they are unable to fall asleep, resulting in no therapy and a possible restless night. Accordingly, there is room for improvement in devices used in providing pressure support therapy and the methods employed thereby.

PCT-application WO2020/136035 discloses a pressure support system for providing pressure support therapy to a patient. The pressure support system includes an airflow generator structured to generate a flow of breathing gas to the patient, a number of sensors structured to sense characteristics of breaths of the patient, and a processing unit structured to calculate a number of breath features of the patient based on the characteristics of breaths of the patient, to calculate a comfort level based on one or more of the calculated number of breath features, and to adjust a gain of the airflow generator based on the calculated comfort level. PCT-application WO2018/115216 A1 discloses a pressure support device for providing pressure support therapy to a patient includes an airflow generator structured to generate pressure to provide pressure compensation to the patient via a patient circuit, one or more sensors structured to gather data related to effectiveness of the pressure compensation, and a processing unit structured to analyze outputs of the sensors while pressure support therapy is provided to the patient to determine if the pressure compensation provided to the patient is improper and to initiate action to confirm whether components of the patient circuit have been changed in response to determining that the pressure compensation provided to the patient is improper.

### SUMMARY OF THE INVENTION

The invention is as defined in the appended claims. Subject-matter referred as embodiments, examples, aspects and/or disclosures which are not covered by the claims are not part of the invention. Embodiments provide improved devices for providing pressure support therapy to patients. As one aspect of the disclosure a method (not claimed) of providing a pressure support therapy to a patient via a PAP device is provided. The method comprises: providing a flow of a breathing gas at a first pressure to an airway of the patient; receiving a first indication from the patient of an adjustment to the first pressure; and responsive to receiving the first indication, providing the flow of breathing gas at a second pressure to the airway of the patient.

Receiving the first indication may comprise receiving a selection of a pressure level from a plurality of predetermined pressure levels.

Receiving the first indication may comprise receiving an indication of an incremental increase or decrease to the first pressure. The incremental increase or decrease to the first pressure may be from 0.5 cmH2O to 2 cmH2O. In an example embodiment, the incremental increase or decrease is 1 cmH2O.

The method may further comprise: receiving a second indication from the patient of an adjustment to the second pressure while providing the flow of breathing gas at the second pressure to the airway of the patient; and responsive to receiving the second indication, providing the flow of breathing gas at a third pressure to the airway of the patient.

The method may further comprise providing an indication of the pressure of the flow of breathing gas to the patient. The indication may comprise one or more of: a numerical value, a graphical display, and/or a letter or combination of letters.

As one aspect of the present invention a pressure support device for providing pressure support therapy to a patient is provided. The pressure support device comprises: an airflow generator structured to generate a flow of breathing gas to provide pressure compensation to the patient via a patient circuit; an input/output arrangement for receiving an input to set parameters of the pressure support device; and a processing unit programmed to cause the airflow generator to provide the flow of a breathing gas at a first pressure for communication to an airway of the patient. The pressure support device is characterized in that the input/output arrangement comprises one or more of an electromechanical button and a touch screen, and the processing unit is programmed to: receive, from the patient via the input/output arrangement pressing the electromechanical button or selecting an icon on the touch screen, a first indication of an adjustment to the first pressure; and responsive to receiving the first indication, provide the flow of breathing gas at a second pressure for communication to the airway of the patient.

The first indication may comprise a selection of a pressure level from a plurality of predetermined pressure levels.

The first indication may comprise an indication of an incremental increase or decrease to the first pressure. The incremental increase or decrease to the first pressure may be from 0.5 cmH2O to 2 cmH2O. In an example embodiment, the incremental increase or decrease is 1 cmH2O.

The processing unit may be further programmed to: receive a second indication of an adjustment to the second pressure while providing the flow of breathing gas at the second pressure; and responsive to receiving the second indication, provide the flow of breathing gas at a third pressure for communication to the airway of the patient.

The pressure support device may further comprise an output arrangement, wherein the processing unit is further programmed to provide an indication of the pressure of the flow of breathing via the output arrangement, and wherein the indication comprises one or more of: a numerical value, a graphical display, and/or a letter or combination of letters.

As another aspect of the present invention, a tangible machine readable medium is provided. The tangible machine readable medium comprises instructions for causing a processing unit of a pressure support device according to an aspect of the invention to: cause the airflow generator to provide the flow of a breathing gas at the first pressure; receive, from the patient via the input/output arrangement pressing the electromechanical button or selecting an icon on the touch screen, the first indication of the adjustment to the first pressure; and adjust with the airflow generator the provided flow of breathing gas to the second pressure responsive to receiving the first.

The machine readable medium may comprise further instructions for causing the airflow generator of the pressure support device to: adjust the provided flow of breathing gas to a third pressure responsive to receiving a second indication from the patient of the third pressure.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1 is a schematic diagram of a pressure support system adapted to provide a regimen of respiratory therapy to a patient according to one example embodiment of the present invention;
FIG. 2 is a schematic diagram of a processing unit according to one example embodiment of the present invention;
FIG. 3 is a flowchart of a method of providing a pressure support therapy to a patient according to one example of the present technology that can be carried out using a pressure support system such as shown in FIG. 1; and
FIG. 4 is a flowchart of another method of providing a pressure support therapy to a patient according to another exampl of the present technology that can be carried out using a pressure support system such as shown in FIG. 1.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

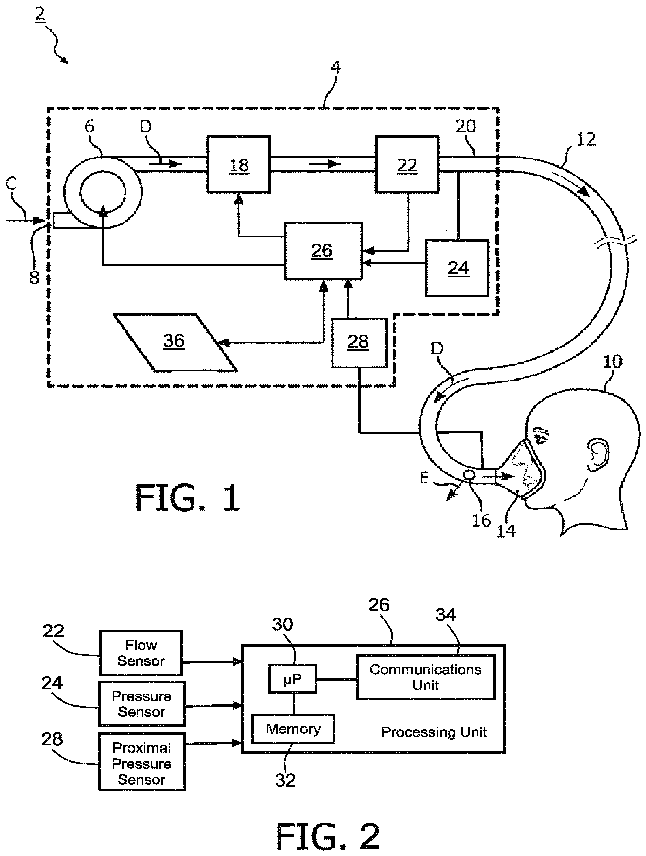

Referring to FIG. 1, a schematic diagram of an airway pressure support system 2 according to one particular, non-limiting exemplary embodiment of the present invention in which methods in accordance with examples of the present disclosure may be implemented is shown. Airway pressure support system 2 includes a pressure support device 4 which houses a gas flow generator 6, such as a blower used in a conventional CPAP or bi-level pressure support device. Gas flow generator 6 receives breathing gas, generally indicated by arrow C, from the ambient atmosphere through an air inlet 8 provided as part of pressure support device 4, and generates a flow of breathing gas therefrom for delivery to an airway of a patient 10 at relatively higher and lower pressures, i.e., generally equal to or above ambient atmospheric pressure. In the example embodiment shown in FIG. 1, gas flow generator 6 is capable of providing a flow of breathing gas ranging in pressure from 3-30 cmH2O, but typically operates at prescription pressures from 4-20 cmH2O. The pressurized flow of breathing gas from gas flow generator 6, generally indicated by arrow D, is delivered via a delivery conduit 12 to a breathing mask or patient interface 14 of any known construction, which is typically worn by, or otherwise attached to, patient 10 in order to communicate flow D of breathing gas to the airway of patient 10. Delivery conduit 12 and patient interface device 14 are typically collectively referred to as a patient circuit.

Pressure support system 2 shown in FIG. 1 is what is known as a singlelimb system, meaning that the patient circuit includes only delivery conduit 12 connecting patient 10 to pressure support system 2. As such, an exhaust vent 16 is provided in delivery conduit 12 for venting exhaled gases from the system as indicated by arrow E. It should be noted that exhaust vent 16 can be provided at other locations in addition to, or instead of in delivery conduit 12, such as in patient interface device 14. It should also be understood that exhaust vent 16 can have a wide variety of configurations depending on the desired manner in which gas is to be vented from pressure support system 2.

The present technology also contemplates that pressure support system 2 can be a two-limb system, having a delivery conduit and an exhaust conduit connected to patient 10. In a two-limb system (also referred to as a dual-limb system), the exhaust conduit carries exhaust gas from patient 10 and includes an exhaust valve at the end distal from patient 10. The exhaust valve in such an embodiment is typically actively controlled to maintain a desired level or pressure in the system, which is commonly known as positive end expiratory pressure (PEEP).

Furthermore, in the example embodiment shown in FIG. 1, patient interface 14 is a nasal/oral mask. It is to be understood, however, that patient interface 14 can include a nasal mask, nasal pillows, a tracheal tube, an endotracheal tube, or any other device that provides a suitable gas flow communicating function. Also, for purposes of the present invention, the phrase "patient interface" can include delivery conduit 12 and any other structures that couple the source of pressurized breathing gas to patient 10.

In the example embodiment shown in FIG. 1, pressure support system 2 includes a pressure controller in the form of a valve 18 provided in an internal delivery conduit 20 provided in a housing (not numbered) of pressure support device 4. Valve 18 controls the pressure of flow D of breathing gas from gas flow generator 6 that is delivered to patient 10. For present purposes, gas flow generator 6 and valve 18 are collectively referred to as a pressure generating system because they act in concert to control the pressure and/or flow of gas delivered to patient 10. However, it should be apparent that other techniques for controlling the pressure of the gas delivered to patient 10, such as varying the blower speed of gas flow generator 6, either alone or in combination with a pressure control valve, are contemplated by the present invention. Thus, valve 18 is optional depending on the technique used to control the pressure of the flow of breathing gas delivered to patient 10. If valve 18 is eliminated, the pressure generating system corresponds to gas flow generator 6 alone, and the pressure of gas in the patient circuit is controlled, for example, by controlling the motor speed of gas flow generator 6.

Pressure support system 2 further includes a flow sensor 22 that measures the flow of the breathing gas within delivery conduit 20 and delivery conduit 12. In the particular embodiment shown in FIG. 1, flow sensor 22 is interposed in line with delivery conduits 20 and 12, most preferably downstream of valve 18. Pressure support system 2 additionally includes a pressure sensor 24 that detects the pressure of the pressurized fluid in delivery conduit 20. While the points at which the flow is measured by flow sensor 22 and the pressure is measured by pressure sensor 24 are illustrated as being within pressure support device 4, it is to be understood that the location at which the actual flow and pressure measurements are taken may be anywhere along delivery conduits 20 or 12. The flow of breathing gas measured by flow sensor 22 and the pressure detected by pressure sensor 24 are provided to a processing unit 26 to determine the flow D of gas to patient 10.

Techniques for calculating the flow D of gas to patient 10 are well known, and take into consideration the pressure drop of the patient circuit, known leaks from the system, i.e., the intentional exhausting of gas from the circuit as indicated by arrow E in FIG. 1, and unknown leaks from the system, such as leaks at the mask/patient interface. The present technology contemplates using any known or hereafter developed technique for calculating leak flow, and using this determination in calculating flow D to patient 10 using measured flow and pressure. Examples of such techniques are taught by U.S. Patent Nos. 5,148,802; 5,313,937; 5,433,193; 5,632,269; 5,803,065; 6,029,664; 6,539,940; 6,626,175; and 7,011,091.

Of course, other techniques for measuring the respiratory flow of patient 10 are contemplated by the present technology, such as, without limitation, measuring the flow directly at patient 10 or at other locations along delivery conduit 12, measuring patient flow based on the operation of gas flow generator 6, and measuring patient flow using a flow sensor upstream of valve 18.

In some non-limiting embodiments of the disclosed concept, pressure support system 2 also includes a proximal pressure sensor 28 that is in fluid communication with a point along delivery conduit 12. For example, without limitation, proximal pressure sensor 28 may be in fluid communication with a point on delivery conduit 12 near patient interface device 14 via a probe connected between proximal pressure sensor 28 and the point on delivery conduit 12. Proximal pressure sensor 28 facilitates measuring pressure proximate the point on delivery conduit 12 and provides the measured proximal pressure to processing unit 24. It will be appreciated that in some exemplary embodiments, proximal pressure sensor 28 may be omitted.

While the flow sensor 22, pressure sensor 24, and proximal pressure sensor 28 have been shown in conjunction with the pressure support system 2 illustrated in FIG. 1, it will be appreciated by those having ordinary skill in the art that other types of sensors may also be employed in conjunction with pressure support system 2 without departing from the scope of the disclosed concept.

Referring now to FIG. 2 in addition to FIG. 1, processing unit 26 includes a processor 30, a memory 32, and a communication unit 34. Processor 30 may form all or part of a processing portion which may be, for example, a microprocessor, a microcontroller any other suitable processing device. Memory 32 may form all or part of a memory portion that may be internal to the processing portion or operatively coupled to the processing portion and provide a storage medium for data and software executable by the processing portion for implementing functionality of processing unit 26 and controlling the operation of pressure support system 2. Memory 32 can be any of one or more of a variety of types of internal and/or external storage media such as, without limitation, RAM, ROM, EPROM(s), EEPROM(s), FLASH, and the like that provide a storage register, i.e., a machine readable medium, for data storage such as in the fashion of an internal storage area of a computer, and can be volatile memory or nonvolatile memory. Processing unit 26 is structured to receive outputs of one or more sensors structured to gather data related to effectiveness of the pressure support therapy and to provide control outputs to one or more elements of pressure support system 2. An example of such sensors is flow sensor 22 and pressure sensor 24, however, other types of sensors may also gather data related to effectiveness of the pressure support therapy and be employed with processing unit 26. Processing unit 26 is also structured to analyze outputs of the sensors while pressure support therapy is provided to the patient to determine patient airflow and pressure waveforms in the patient circuit.

An input/output arrangement 36 is provided for setting various parameters used by airway pressure support system 2, as well as for displaying and outputting information and data to a user, such as a patient, clinician or caregiver. Input/output arrangement 36 may include one or more of: a display, electromechanical buttons, a touchscreen, or any other suitable arrangement for providing input to, or providing output from, processing unit 26 and may be included in/on the housing of pressure support device 4 or separate therefrom and in wireless communication (e.g., via Bluetooth^{®} or other suitable arrangement) with processing unit 26.

It will be appreciated that pressure support device 4 may include additional components that are not illustrated in the schematic diagram of FIG. 1. For example, without limitation, pressure support device 4 may include a filter to filter breathing gas provided to patient 10 and/or a humidifier to humidify breathing gas provided to patient 10.

In the illustrated, non-limiting exemplary embodiment of the present technology, airway pressure support system 2 essentially functions as a CPAP pressure support system and pressure support device 4 provides functions of a CPAP base unit. Pressure support system 2, therefore, includes all of the capabilities necessary in such systems in order to provide appropriate CPAP pressure levels to patient 10. This includes receiving the necessary parameters, via input commands, signals, instructions or other information, for providing appropriate CPAP pressure, such as maximum and minimum CPAP pressure settings. It should be understood that this is meant to be exemplary only, and that other pressure support methodologies, including, but not limited to, BiPAP AutoSV, AVAPS, Auto CPAP, and BiPAP Auto, are within the scope of the present invention.

Communication unit 34 may provide for communication between processing unit 26 and other components of pressure support device 4, components of the patient circuit, or other external devices via the internet, cellular, WiFi, wired telephone line, or any other suitable means. For example, without limitation, communication unit 34 may facilitate communication with various sensors such as flow control sensor 22. Communication unit 34 may also facilitate communication with external devices. For example, without limitation, communication unit 34 may facilitate communication with electronic devices such as a phone, tablet, computer, or other devices whether local or distant, directly or via a network. Communication facilitated by communication unit 34 may allow processing unit 26 to send and/or receive data from the component or device it communicates with.

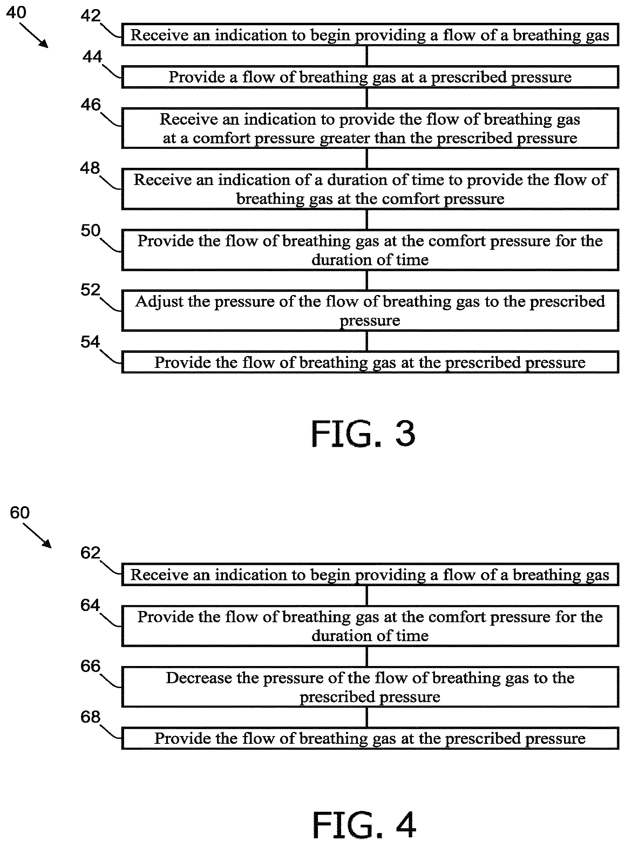

Having thus described the basic arrangement and components of pressure support system 2 and pressure support device 4 thereof, a method 40 in accordance with one example which can be carried out using pressure support device 4 will now be described in conjunction with the flow chart of FIG. 3. Such method is an example of what would typically be carried out when a patient uses a pressure support device 4, in accordance with an embodiment of the present invention, for the first time. As shown at 42, method 40 begins when an indication to begin providing a flow of a breathing gas is received by processing unit 26. Such indication may be provided by a user (i.e., a patient): powering up pressure support device 4 via a power button or other suitable arrangement, by selecting a "start" function such a via input/output arrangement 36, or via any other suitable arrangement. After such indication is received, processing unit 26 causes pressure support device 4 to begin to provide flow D of breathing gas at a pressure prescribed to the patient by a caregiver (i.e., a prescribed pressure), such as shown at 44. If/when an indication to provide the flow of breathing gas at a comfort pressure other than the prescribed pressure is received by processing unit 26 (e.g., greater or lesser than the prescribed pressure), such as shown at 46, pressure support device then provides the flow of breathing gas at the comfort pressure, such as shown at 50. Such indication is provided by a user pressing a button or selecting an icon on a touchscreen.

The comfort pressure may be a preset pressure selected/set by a manufacturer, caregiver or by the user, or may be a pressure selected by the user (e.g., via input/output arrangement 36 after turning on pressure support device 4. Unlike conventional arrangements such as previously discussed in the Background section herein, the selection of the comfort pressure by the patient may be made while pressure support device 4 is providing flow D of breathing gas to the patient, thus allowing for the patient to actively feel the selected comfort pressure being provided by pressure support device 4, and adjustments made thereto, and decide upon the pressure that feels best to the patient. In one example, the user/patient is provided with selectable options for setting the comfort pressure, e.g., low, medium, high, max wherein each setting corresponds to a particular pressure (e.g., low - 4 cmH2O, med - 6 cH2O, high - 8 cm H2O, max - 10 cm H2O). In other example embodiments of the present invention, the user is able to select the comfort pressure with greater granularity either by increasing/decreasing a selected comfort pressure in small increments (e.g., 0.5 cmH2O, 1 cmH2O, 1.5 cnH2O, 2 cmH2O, or any other suitable increment) or by directly inputting a desired comfort pressure value from within an allowed range of comfort pressure values (e.g., 4-10 cmH2O). In any case, unlike conventional arrangements such as previously discussed in which the ramp pressure was limited to a pressure less than the prescribed pressure, the comfort pressure selected by the patient can exceed the prescribed pressure. In examples, indications indications of the comfort pressure are provided to the user, such as via input/output arrangement 36. Such indications may be in the form of numerical values (e.g., without limitation, "4 cmH2O", "4",etc.), graphical displays (e.g., without limitation, 1 bar, 2 bars, 3 bars, etc.), letters or combinations of letters (e.g., without limitation, "L", "M", "H", "Low", "Medium", "High", etc.) or any other suitable indicator(s) of the pressure or relative pressure level of the comfort pressure provided to the patient.

Continuing to refer to FIG. 3, as further shown at 50, the flow of breathing gas is provided at the previously discussed comfort pressure for a duration of time. Ideally the duration of time at which the comfort pressure is provided should be slightly longer (e.g., without limitation, 5-10 minutes) than the time it takes the patient to fall asleep. The duration of time may be a preset duration previously set by a manufacturer, caregiver, or the patient themselves, or may be provided/selected (e.g., via input/output arrangement 36) by the patient immediately before or after starting the comfort function, such as shown at 48. In one example, the user/patient is provided with selectable options for setting the duration, e.g., 15 min, 30 min, 45 min. In other example embodiments the user can adjust the duration with greater granularity either by increasing/decreasing the duration in predetermined increments (e.g., 1 min, 5 min) or by directly inputting a desired duration value from within an allowed range of duration values (e.g., without limitation, 10-60 min). Once the patient selects/sets the comfortable starting pressure, this pressure will be constantly delivered to the patient for the selected/set duration of time without increasing or ramping. However, in one example embodiment, if a patient falls asleep during the selected period and experiences an event, an auto algorithm will kick in (similar to SMART Ramp previously discussed) and will adjust and deliver pressure to the patient accordingly to what is needed in order to avoid additional events. Overall the general idea is that if the selected pressure is what is most comfortable for a patient, such pressure should be delivered to the patient until they fall asleep without making any changes that may cause the patient to not fall asleep. Once the duration of time has passed, the pressure of the provided flow of breathing gas is adjusted (i.e., increased or decreased) to the prescribed pressure(s) and pressure support device 4 will go on to deliver the appropriate therapy/prescribed pressures, such as shown at 52 and 54. In examples, such adjustment between the comfort pressure and the prescribed pressure may occur in a linear fashion over a period of time (e.g., a 1-5 minutes) or via any other suitable manner that minimizes potential disturbance of the patient.

Another method 60 in accordance with another example of the present technology which can be carried out using pressure support device 4 will now be described in conjunction with the flow chart of FIG. 4. Such method is generally an example of what would typically be carried out when a patient uses a pressure support device 4 in accordance with an exampled **of** the present technology subsequent to the usage described in conjunction with the method of FIG. 3. As shown at 62, method 60 begins when an indication to begin providing a flow of a breathing gas is received by processing unit 26. As previously discussed in conjunction with 42, such indication may be provided by a user (i.e., a patient): powering up pressure support device 4 via a power button or other suitable arrangement, by selecting a "start" function such a via input/output arrangement 36, or via any other suitable arrangement. As shown at 64, after such indication is received at 62, processing unit 26 causes pressure support device 4 to begin to provide flow D of breathing gas at a comfort pressure, such as previously discussed, that is different than a prescribed pressure since such comfort pressure was previously indicated (such as via method 40) as being a desirable startup pressure at which to receive the flow of breathing gas until the patient falls asleep. As further shown at 64, the flow of breathing gas is provided constantly at the previously discussed comfort pressure for a duration of time, which may also be a duration previously selected by the patient such as previously described in conjunction with method 40 or at a new duration selected by the patient selected in a manner such as previously described. As discussed in regard to method 40, such comfort pressure will be constantly delivered to the patient for the selected/set duration of time without increasing or ramping. However, in one example embodiment, if a patient falls asleep during the selected period and experiences an event, an auto algorithm will kick in (like in SMART Ramp previously discussed) and will adjust and deliver pressure to the patient accordingly to what is needed in order to avoid additional events. Once again, the general idea is that if the selected pressure is what is most comfortable for a patient, such pressure should be delivered to the patient until they fall asleep without making any changes that may cause the patient to not fall asleep. Once the duration of time has passed, the pressure of the provided flow of breathing gas is adjusted to the prescribed pressure(s) and pressure support device 4 will go on to deliver the appropriate therapy/prescribed pressures, such as shown at 66 and 68. As previously discussed in regard to method 40, in examples of the present technology, such adjustment (whether an increase or decrease in pressure) between the comfort pressure and the prescribed pressure may occur in a linear fashion over a period of time (e.g., a 1-5 minutes) or via any other suitable manner that minimizes potential disturbance of the patient.

From the foregoing it is thus to be appreciated that embodiments of the present invention provide arrangements that improve the comfort of patient receiving pressure support therapy as compared to conventional solutions.

It is contemplated that aspects of the disclosed concept can be embodied as computer readable codes on a tangible computer readable recording medium. The computer readable recording medium is any data storage device that can store data which can be thereafter read by a computer system. Examples of the computer readable recording medium include read-only memory (ROM), random-access memory (RAM), CD-ROMs, magnetic tapes, floppy disks, and optical data storage devices.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

## Claims

1. A pressure support device (2) for providing pressure support therapy to a patient, the pressure support device comprising:
an airflow generator (6) structured to generate a flow of breathing gas to provide pressure compensation to the patient via a patient circuit;
an input/output arrangement (36) for receiving an input to set parameters of the pressure support device; and
a processing unit (26) programmed to cause the airflow generator to provide the flow of a breathing gas at a first pressure for communication to an airway of the patient;
wherein the input/output arrangement (36) comprises one or more of an electromechanical button and a touch screen, and
wherein the processing unit (26) is programmed to:
receive, from the patient via the input/output arrangement pressing the electromechanical button or selecting an icon on the touch screen, a first indication of an adjustment to the first pressure; and
responsive to receiving the first indication, provide the flow of breathing gas at a second pressure for communication to the airway of the patient.

2. The pressure support device (2) of claim 1, wherein the first indication comprises a selection of a pressure level from a plurality of predetermined pressure levels.

3. The pressure support device (2) of claim 1, wherein the first indication comprises an indication of an incremental increase or decrease to the first pressure.

4. The pressure support device (2) of claim 3, wherein the incremental increase or decrease to the first pressure is a 1 cmH2O increase or decrease.

5. The pressure support device (2) of claim 1, wherein the processing unit (26) is further programmed to:
receive a second indication of an adjustment to the second pressure while providing the flow of breathing gas at the second pressure; and
responsive to receiving the second indication, provide the flow of breathing gas at a third pressure for communication to the airway of the patient.

6. The pressure support device (2) of claim 1, wherein the processing unit (26) is further programmed to provide an indication of the pressure of the flow of breathing via the input/output arrangement, and wherein the indication comprises one or more of: a numerical value, a graphical display, and/or a letter or combination of letters.

7. A tangible machine readable medium comprising instructions for causing a processing unit (26) of a pressure support device according to any one of claims 1-6 to:
cause the airflow generator (6) to provide the flow of a breathing gas at the first pressure;
receive, from the patient via the input/output arrangement pressing the electromechanical button or selecting an icon on the touch screen, the first indication of the adjustment to the first pressure; and
adjust with the airflow generator the provided flow of breathing gas to the second pressure responsive to receiving the first indication.

8. The machine readable medium of claim 7, comprising further instructions for causing the airflow generator of the pressure support device to:
adjust the provided flow of breathing gas to a third pressure responsive to receiving a second indication from the patient of the third pressure.

## Patentansprüche

1. Druckunterstützungsvorrichtung (2) zum Bereitstellen einer Druckunterstützungstherapie für einen
Patienten, die Druckunterstützungsvorrichtung umfassend:
einen Luftstromgenerator (6), der strukturiert ist, um einen Atemgasstrom zu generieren, um dem Patienten über einen Patientenkreislauf einen Druckausgleich bereitzustellen;
eine Eingangs-/Ausgangsanordnung (36) zum Empfangen eines Eingangs, um Parameter der Druckunterstützungsvorrichtung einzustellen; und
eine Verarbeitungseinheit (26), die programmiert ist, um den Luftstromgenerator zu veranlassen, den Strom eines Atemgases an einem ersten Druck zur Verbindung mit einem Atemweg des Patienten bereitzustellen;
wobei die Eingangs-/Ausgangsanordnung (36) eines oder mehrere einer elektromechanischen Taste und eines Berührungsbildschirms umfasst,
wobei die Verarbeitungseinheit (26) programmiert ist, um:
vom Patienten über die Eingangs-/Ausgangsanordnung durch Drücken der elektromechanischen Taste oder Auswählen eines Symbols auf dem Berührungsbildschirm eine erste Angabe einer Anpassung auf den ersten Druck zu empfangen; und
als Reaktion auf Empfangen der ersten Angabe den Atemgasstrom an einem zweiten Druck zur Verbindung mit dem Atemweg des Patienten bereitzustellen.

2. Druckunterstützungsvorrichtung (2) nach Anspruch 1, wobei die erste Angabe eine Auswahl einer Druckstufe aus einer Vielzahl vorbestimmter Druckstufen umfasst.

3. Druckunterstützungsvorrichtung (2) nach Anspruch 1, wobei die erste Angabe eine Angabe einer inkrementalen Erhöhung oder Verringerung auf den ersten Druck umfasst.

4. Druckunterstützungsvorrichtung (2) nach Anspruch 3, wobei die inkrementale Erhöhung oder Verringerung auf den ersten Druck eine Erhöhung oder Verringerung um 1 cmH20 ist.

5. Druckunterstützungsvorrichtung (2) nach Anspruch 1, wobei die Verarbeitungseinheit (26) weiter programmiert ist, um:
eine zweite Angabe einer Anpassung auf den zweiten Druck zu empfangen, während der Atemgasstrom am zweiten Druck bereitgestellt wird; und
als Reaktion auf Empfangen der zweiten Angabe den Atemgasstrom an einem dritten Druck zur Verbindung mit dem Atemweg des Patienten bereitzustellen.

6. Druckunterstützungsvorrichtung (2) nach Anspruch 1, wobei die Verarbeitungseinheit (26) weiter programmiert ist, um eine Angabe des Drucks des Atemstroms über die Eingangs-/Ausgangsanordnung bereitzustellen, wobei die Angabe eines oder mehr umfasst von: einem Zahlenwert, einer grafischen Darstellung und/oder einem Buchstaben oder einer Buchstabenkombination.

7. Greifbares maschinenlesbares Medium, umfassend Anweisungen, die eine Verarbeitungseinheit (26) einer Druckunterstützungsvorrichtung nach einem der Ansprüche 1-6 veranlassen:
den Luftstromgenerator (6) zu veranlassen, den Atemgasstrom am ersten Druck bereitzustellen;
vom Patienten über die Eingangs-/Ausgangsanordnung durch Drücken der elektromechanischen Taste oder Auswählen eines Symbols auf dem Berührungsbildschirm die erste Angabe der Anpassung auf den ersten Druck zu empfangen; und
mit dem Luftstromgenerator als Reaktion auf Empfangen der ersten Angabe den bereitgestellten Atemgasstrom auf den zweiten Druck anzupassen.

8. Maschinenlesbares Medium nach Anspruch 7, umfassend weitere Anweisungen zum Veranlassen des Luftstromgenerators der Druckunterstützungsvorrichtung:
zum Anpassen des bereitgestellten Atemgasstroms auf einen dritten Druck als Reaktion auf Empfangen einer zweiten Angabe des dritten Drucks vom Patienten.

## Revendications

1. Dispositif d'aide inspiratoire (2) destiné à fournir une thérapie d'aide inspiratoire à un
patient, le dispositif d'aide inspiratoire comprenant :
un générateur de flux d'air (6) structuré pour générer un flux de gaz respiratoire pour fournir une compensation de pression au patient via un circuit patient ;
un agencement d'entrée/de sortie (36) pour recevoir une entrée pour régler des paramètres du dispositif d'aide inspiratoire ; et
une unité de traitement (26) programmée pour amener le générateur de flux d'air à fournir le flux d'un gaz respiratoire à une première pression pour une communication avec les voies respiratoires du patient ;
dans lequel l'agencement d'entrée/de sortie (36) comprend un ou plusieurs d'un bouton électromécanique et d'un écran tactile, et
dans lequel l'unité de traitement (26) est programmée pour :
recevoir, du patient, via l'agencement d'entrée/de sortie, appuyant sur le bouton électromécanique ou sélectionnant une icône sur l'écran tactile, une première indication d'un ajustement à la première pression ; et
à la suite de la première indication, fournir un débit de gaz respiratoire à une deuxième pression pour une communication avec les voies respiratoires du patient.

2. Dispositif d'aide inspiratoire (2) selon la revendication 1, dans lequel la première indication comprend une sélection d'un niveau de pression parmi une pluralité de niveaux de pression prédéterminés.

3. Dispositif d'aide inspiratoire (2) selon la revendication 1, dans lequel la première indication comprend une indication d'une augmentation ou d'une diminution croissante de la première pression.

4. Dispositif d'aide inspiratoire (2) selon la revendication 3, dans lequel l'augmentation ou la diminution croissante de la première pression est une augmentation ou une diminution de 1 cmH20.

5. Dispositif d'aide inspiratoire (2) selon la revendication 1, dans lequel l'unité de traitement (26) est en outre programmée pour :
recevoir une seconde indication d'un ajustement de la deuxième pression tout en fournissant le débit de gaz respiratoire à la deuxième pression ; et
à la suite de la réception de la seconde indication, fournir le débit de gaz respiratoire à une troisième pression pour une communication avec les voies respiratoires du patient.

6. Dispositif d'aide inspiratoire (2) selon la revendication 1, dans lequel l'unité de traitement (26) est en outre programmée pour fournir une indication de la pression du flux respiratoire via l'agencement d'entrée/de sortie et dans lequel l'indication comprend un ou plusieurs : d'une valeur numérique, d'un affichage graphique et/ou d'une lettre ou d'une combinaison de lettres.

7. Support tangible lisible par machine comprenant des instructions pour amener une unité de traitement (26) d'un dispositif d'aide inspiratoire selon l'une quelconque des revendications 1-6 à :
amener le générateur de flux d'air (6) à fournir le flux d'un gaz respiratoire à la première pression ;
recevoir, du patient via l'agencement d'entrée/de sortie, appuyant sur le bouton électromécanique ou sélectionnant une icône sur l'écran tactile, la première indication de l'ajustement à la première pression ; et
ajuster, avec le générateur de flux d'air, le débit fourni de gaz respiratoire à la deuxième pression à la suite de la réception de la première indication.

8. Support lisible par machine selon la revendication 7, comprenant des instructions supplémentaires pour amener le générateur de flux d'air du dispositif d'aide inspiratoire à :
ajuster le débit fourni de gaz respiratoire à une troisième pression à la suite de la réception d'une seconde indication du patient de la troisième pression.
